# EUROPEAN PATENT SPECIFICATION

(11) **EP 2 517 739 B1**
(45) Date of publication and mention of the grant of the patent: **25.12.2013**
(21) Application number: 12157208.5
(22) Date of filing: 28.02.2012
(51) Int. Cl.: A61M 1/10, A61M 1/12

(54) **Blood pump, in particular implantable pneumatic heart assist device**
Blutpumpe, insbesondere eine pneumatische Herzunterstützungsvorrichtung
Pompe sanguine, en particulier un dispositif d'assistance cardiaque pneumatique

(30) Priority: 28.02.2011 PL 39405811
(43) Date of publication of application: 31.10.2012
(73) Proprietor: Fundacja Rozwoju Kardiochirurgii Im. Prof. Zbigniewa Religi, 41-800 Zabrze (PL)
(72) Inventor: Darlak, Maciej, 41-710 Ruda Slaska (PL); Kustosz, Roman, 41-800 Zabrze (PL); Kapis, Artur, 41-800 Zabrze (PL)
(74) Representative: Malcherek, Piotr

(56) References cited:
- WO-A1-90/07648
- WO-A1-2008/012007
- WO-A1-2009/118499
- CN-Y- 201 370 810
- US-A- 5 089 016

## Description

The subject of the invention is a blood pump, in particular an implantable pneumatic ventricular assist device.

Pneumatic ventricular assist devices are applied to support the heart functions in patients suffering from failure of the cardiovascular system. The known ventricular assist device, which is a blood pump in practice, comprises a spherical cap including an air chamber and a blood chamber separated by a membrane. The membrane is mounted around the internal circumference of the cap, wherein said circumference is the base of the blood chamber. The blood chamber has an inlet channel and an outlet channel for blood, wherein said channels have circular seats for mounting valves for regulating the blood flow through the pump. For each seat there is an axis, perpendicular to the plane defined by the clearance of said seat, inclined at an angle towards the spherical cap of the pump in relation to the plane defined by the base of the blood chamber. In the structure of the ventricular assist device devised by the Prof. Zbigniew Religa's Foundation for Cardiac Surgery Development the axes perpendicular to the plane defined by the clearance of the seats are mutually parallel and inclined towards the spherical cap of the pump at an angle 15° in relation to the plane defined by the base of the blood chamber. Connectors for linking the pump to cannulas connecting the pump to the circulatory system of a patient are fixed to the inlet and outlet channels with first ends thereof. The air chamber of the spherical cap is connected to a generator generating an alternate pneumatic wave which stimulates alternate movement of the membrane located between the air chamber of the spherical cap and the blood chamber thereof. The alternate movement of the membrane results in the blood flowing through the device.

An example of a pneumatic ventricular assist device is known from the Polish utility model description PL63283Y. Other similar pumps are known, for example, from the following patent descriptions CN201370810, WO2008/012007, DE 10217635 or US 6183220.

The invention as claimed relates to a blood pump, in particular an implantable pneumatic ventricular assist device which comprises a spherical cap with an air chamber and a blood chamber. Inside the spherical cap around the circumference thereof there is a membrane which separates the air chamber of the spherical cap from the blood chamber of the spherical cap. The place of mounting the membrane around the inside circumference of the spherical cap is the base of the blood chamber. Additionally, the blood chamber of the spherical cap has an inlet channel for blood and an outlet channel for blood, and in each of said channels there is a circular seat for mounting a valve therein in order to regulate the blood flow through the pump, wherein for each of the seats the axis perpendicular to the surface determined by the clearance of said seat is inclined at an angle in the direction of the spherical cap to the plane determined by the base of the blood chamber. The essence of the invention as claimed is that the axis of the seat of the inlet channel is inclined towards the plane determined by the base of the blood chamber at an angle smaller than 10°, whereas the axis of the outlet channel is inclined towards the plane determined by the base of the blood chamber at an angle within the range between 30 ° and 40 °. Moreover, in the projection onto a plane perpendicular to the plane determined by the base of the blood chamber and perpendicular to the projection of the axis of the seat of the inlet channel onto the plane determined by the base of the blood chamber, the axis of the seat of the outlet channel is inclined towards the axis of the seat of the inlet channel at an angle within the range between 22° and 32°. Furthermore, in the projection onto the plane determined by the base of the blood chamber, the axis of the seat of the outlet channel is inclined towards the axis of the seat of the inlet channel at an angle within the range between 15° and 25°.

Preferably, the extreme line which determines the run of the inlet channel is tangent to the circumference of the inside of the blood chamber of the spherical cap, whereas the outlet channel has the form of a non-symmetrical funnel narrowing towards the seat of the outlet channel.

The axis of the seat of the inlet channel in the described embodiment is inclined towards the plane determined by the base of the blood chamber at an angle smaller than 4°, most preferably 2°.

The axis of the seat of the outlet channel in a preferred embodiment is inclined towards the plane determined by the base of the blood chamber at an angle within the range between 34° and 37°, most preferably 35°.

In further preferred embodiment, in the projection onto the plane perpendicular to the plane determined by the base of the blood chamber and perpendicular to the projection of the axis of the seat of the inlet channel onto the plane determined by the base of the blood chamber the axis of the seat of the outlet channel is inclined towards the axis of the seat of the inlet channel at an angle within the range between 25° and 29°, most preferably 27°.

In another preferred embodiment in the projection onto the plane determined by the base of the blood chamber the axis of the seat of the outlet channel is inclined towards the axis of the seat of the inlet channel at an angle within the range between 18 ° and 22 °, most preferably 20°.

A blood pump, in particular an implantable pneumatic ventricular assist device according to the invention as claimed optimizes the parameters of blood flow in the ventricular assist device owing to the proposed oblique spatial location of the axes of the seats of the inlet and outlet channels. As a result the valves are closer to the spherical surface of the spherical cap and the height and size of the whole ventricular assist device have been reduced, the passages between the inside surfaces of the device and the inlet and outlet channels have been smoothened. As a consequence, the risk of thrombosis is reduced. The new structure enables a convenient connection of the ventricular assist device to the patient's circulatory system during the implantation.

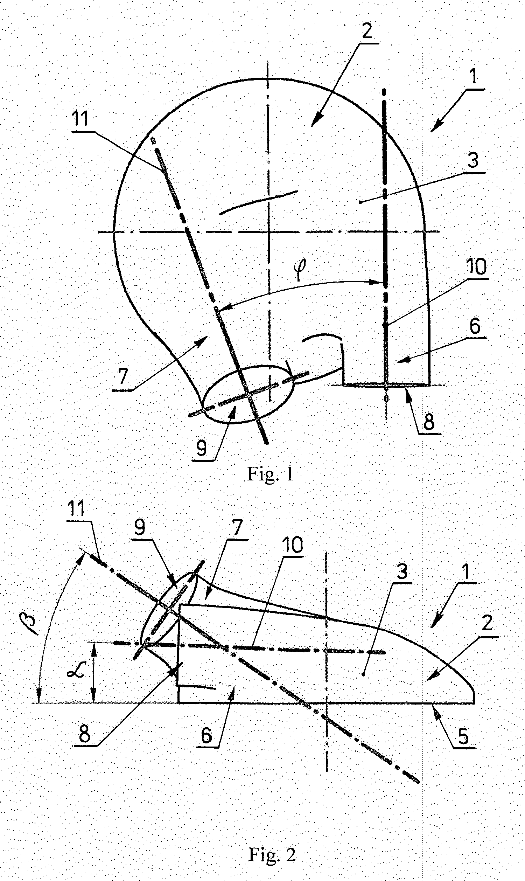

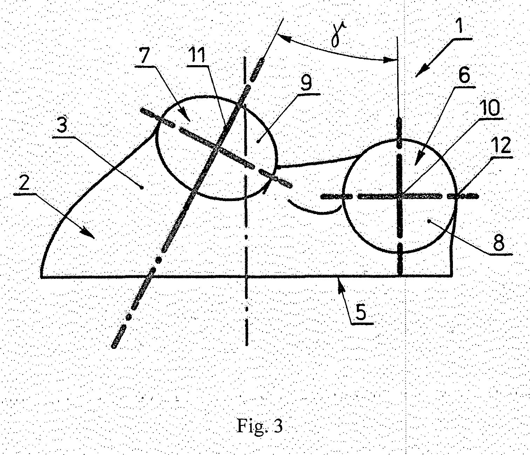

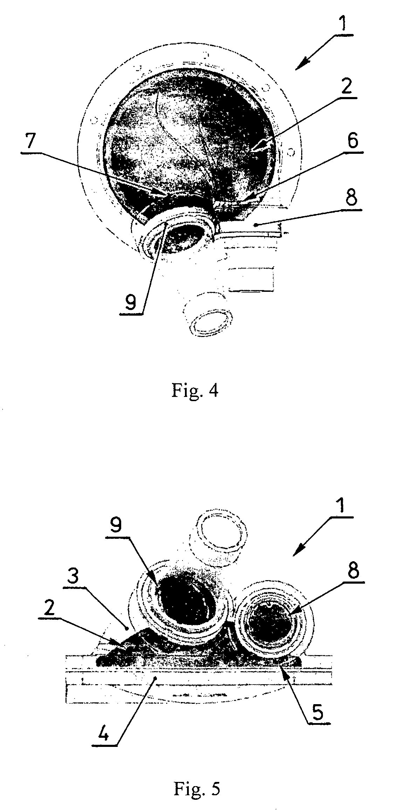

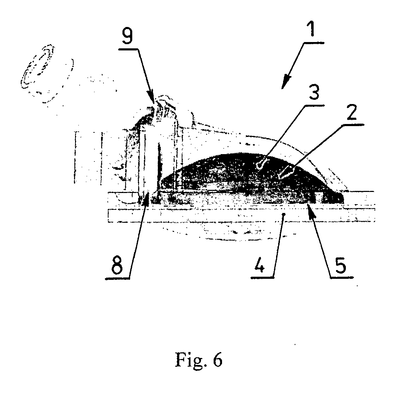

The invention is presented in greater detail in the following embodiment and the enclosed drawing, where fig. 1 is a schematic outline of the blood chamber of the device in the projection onto the plane determined by the base of the blood chamber, fig. 2 - is a schematic outline of the blood chamber of the ventricular assist device in side view, fig. 3 - is a schematic outline of the blood chamber of the device in the projection onto the plane perpendicular to the plane determined by the base of the blood chamber and perpendicular to the projection of the axis of the seat of the inlet channel onto the plane determined by base of the blood chamber, fig. 4 - is a top view of the device, fig. 5 and fig. 6 - is a side view of the ventricle in different positions.

The blood pump has the form of an implantable pneumatic ventricular assist device 1, comprising a spherical cap 2 which has a blood chamber 3 and an air chamber 4. Inside the spherical cap 2 around the inside circumference thereof there is a membrane which separates the air chamber 4 of the spherical cap 2 from the blood chamber 3. The place of mounting the membrane around the inside circumference of the spherical cap 2 determines the base 5 of the blood chamber 3. Additionally, the blood chamber 3 of the spherical cap 2 has an inlet channel 6 for blood and an outlet channel 7 for blood, and in each of the channels 6,7 there is a circular seat 8,9 for mounting therein a heart valve for regulating the blood flow by the pump 1. An axis 10 of the seat 8 of the inlet channel 6, which is perpendicular to the plane determined by the clearance of said seat 8, corresponding with the location of the heart valve, is inclined towards the plane determined by the base 5 of the blood chamber 3 at an angle α of 2°.

In alternative embodiments of the invention the angle may range below 10°, for example 4°.

An axis 11 of the seat 9 of the outlet channel 7, which is perpendicular to the plane determined by the clearance of said seat 9, corresponding to the location of the heart valve, is inclined towards the plane determined by base 5 of the blood chamber 3 at an angle β of 35°.

In alternative embodiments of the invention the angle β may be within the range between 30° and 40°, most preferably between 34 ° and 37 °.

Moreover, in the projection onto the plane perpendicular to the plane determined by the base 5 of the blood chamber 3 and perpendicular to the projection of the axis 10 of the seat 8 of the inlet channel 6 onto the plane determined by the base 5 of the blood chamber 3 the axis 11 of the seat 9 of the outlet channel 7 is inclined towards the axis 10 of the seat 8 of the inlet channel 6 at an angle γ of 27°.

In alternative embodiments of the invention the angle γ may be within the range between 22° and 32°, most preferably from 25° to 29°.

Furthermore, in the projection onto the plane determined by the base 5 of the blood chamber 3 the axis 11 of the seat 9 of the outlet channel 7 is inclined towards the axis 10 of the seat 8 of the inlet channel 6 at an angle ϕ of 20°.

In alternative embodiments of the invention the angle ϕ may be within the range between 15° and 25°, most preferably between 18° and 22°.

The extreme line 12 which determines the run of the inlet channel 6 is tangent to the circumference of the inside of the blood chamber 3 of the spherical cap, whereas the outlet channel 7 has the form of a non-symmetrical funnel narrowing towards the seat 9 of the outlet channel 7.

## Claims

1. A blood pump, in particular an implantable pneumatic ventricular assist device ( 1 ), with a spherical cap ( 2 ) comprising an air chamber ( 4 ) and a blood chamber ( 3 ), separated by a membrane mounted around the inside circumference of the spherical cap ( 2 ), wherein said circumference determines a base ( 5 ) of the blood chamber ( 3 ), wherein the blood chamber ( 3 ) of the spherical cap ( 2 ) has an inlet channel ( 6 ) for blood and an outlet channel ( 7 ) for blood, and in each of the channels ( 6, 7 ) there is a circular seat ( 8, 9 ) for mounting a heart valve therein to regulate the blood flow through the pump ( 1 ), wherein for each of the seats ( 8, 9 ) the axis ( 10, 11 ) thereof perpendicular to the surface determined by the clearance of said seat ( 8, 9 ) is inclined at an angle towards the spherical cap ( 2 ) of the pump ( 1 ) to the plane determined by the base ( 5 ) of the blood chamber ( 3 ), **characterized in that** the axis ( 10 ) of the seat ( 8 ) of the inlet channel ( 6 ) is inclined to the plane determined by the base ( 5 ) of the blood chamber ( 3 ) at an angle ( a ) smaller than 10°, whereas the axis ( 11 ) of the seat ( 9 ) of the outlet channel ( 7 ) is inclined to the plane determined by the base ( 5 ) of the blood chamber ( 3 ) at an angle ( β ) within the range between 30° and 40°, and in the projection onto the plane perpendicular to the plane determined by the base ( 5 ) of the blood chamber ( 3 ) and perpendicular to the projection of the axis ( 10 ) of the seat ( 8 ) of the inlet channel ( 6 ) onto the plane determined by the base ( 5 ) of the blood chamber ( 3 ) the axis ( 11 ) of the seat ( 9 ) of the outlet chamber ( 7 ) is inclined to the axis ( 10 ) of the seat ( 8 ) of the inlet channel ( 6 ) at an angle ( γ ) within the range between 22° and 32°, and in the projection onto the plane determined by the base ( 5 ) of the blood chamber ( 3 ) the axis ( 11 ) of the seat ( 9 ) of the outlet chamber ( 7 ) is inclined towards the axis ( 10 ) of the seat ( 8 ) of the inlet channel ( 6 ) at an angle ( ϕ ) within the range between 15° and 25°.

2. The pump according to claim 1 **characterized in that** the extreme line ( 12 ) determining the run of the inlet channel ( 6 ) is tangent to the circumference of the inside of the blood chamber ( 3 ) of the spherical cap, and the outlet channel ( 7 ) has a form of an asymmetrical funnel narrowing towards the seat ( 9 ) of the outlet channel ( 7 ).

3. The pump according to claim 1 or 2 **characterized in that** the axis ( 10 ) of the seat ( 8 ) of the inlet channel ( 6 ) is inclined to the plane determined by the base ( 5 ) of the blood chamber ( 3 ) at an angle ( α ) smaller than 4°, preferably 2°.

4. The pump according to one of the above claims **characterized in that** the axis ( 11 ) of the seat ( 9 ) of the outlet channel ( 7 ) is inclined to the plane determined by the base ( 5 ) of the blood chamber ( 3 ) at an angle ( β ) within the range between 34° and 37°, most preferably 35°.

5. The pump according to one of the above claims **characterized in that** in the projection onto the plane perpendicular to the plane determined by the base ( 5 ) of the blood chamber ( 3 ) and perpendicular to the projection of the axis ( 10 ) of the seat ( 8 ) of the inlet channel ( 6 ) onto the plane determined by the base ( 5 ) of the blood chamber ( 3 ) the axis ( 11 ) of the seat ( 9 ) of the outlet channel ( 7 ) is inclined to the axis ( 10 ) of the seat ( 8 ) of the inlet channel ( 6 ) at an angle ( γ ) within the range between 25° and 29°, most preferably 27°.

6. The pump according to one of the above claims **characterized in that** in the projection onto the plane determined by the base ( 5 ) of the blood chamber ( 3 ) the axis ( 11 ) of the seat ( 9 ) of the outlet channel ( 7 ) is inclined to the axis ( 10 ) of the seat ( 8 ) of the inlet channel ( 6 ) at an angle ( ϕ ) within the range between 18 ° and 22 °, most preferably 20°.

## Patentansprüche

1. Blutpumpe, insbesondere eine implantierbare pneumatische Herz-Hilfspumpe /1/ ausgerüstet mit der Schale /2/, die die Luftkammer /4/ und die Blutkammer /3/ aufweist, welche mittels am inneren Umfang der Schale /2/ befestigten Membran voneinander getrennt sind, und die Basis /5/ der Blutkammer /3/ von diesem Umfang festgelegt ist, wobei die Blutkammer /3/ der Schale /2/ mit dem Blut-Einlasskanal /6/ und mit dem Blut-Auslasskanal /7/ ausgerüstet ist, und in jedem der Blut-Kanäle /6,7/ die runde Aufnahmebüchse /8, 9/ für Aufnahme einer Drosselklappe zur Regellung des Blutdurchflusses durch die Blutpumpe /1/ vorgesehen ist, wobei die Achse /10, 11/ jeder Aufnahmebüchse /8, 9/, senkrecht zur durch den lichten Durchmesser dieser Aufnahmebüchse /8, 9/ bestimmten Ebene, ist auf die Schale /2/ der Blutpumpe /1/ hin zu der durch die Basis /5/ der Blutkammer /3/ bestimmten Ebene geneigt, **gekennzeichnet dadurch, dass** die Achse /10/ der Aufnahmebüchse /8/ des Blut-Einlasskanals /6/ zu der durch die Basis /5/ der Blutkammer /3/ bestimmten Ebene unterm Neigungswinkel /α/ von weniger als 10° verläuft, hingegen aber die Achse /11/ der Aufnahmebüchse /9/ des Blut-Auslasskanals /7/ zu der durch die Basis /5/ der Blutkammer /3/ bestimmten Ebene unterm Neigungswinkel /β/ aus dem Bereich von 30° bis 40° verläuft, jedoch im Aufriss auf die Senkrechte zu der durch die Basis /5/ der Blutkammer /3/ bestimmten Ebene sowie senkrechten zum Aufriss der Achse /10/ der Aufnahmebüchse /8/ des Blut-Einlasskanals /6/ auf die durch die Basis /5/ der Blutkammer /3/ bestimmte Ebene verläuft die Achse /11/ der Aufnahmebüchse /9/ des Blut-Auslasskanals /7/ zu der Achse /10/ der Aufnahmebüchse /8/ des Blut-Einlasskanals /6/ unterm Neigungswinkel /γ/ aus dem Bereich von 22° bis 32°, und im Aufriss auf die durch die Basis /5/ der Blutkammer /3/ bestimmte Ebene ist die Achse /11/ der Aufnahmebüchse /9/ des Blut-Auslasskanals /8/ zur Achse /10/ der Aufnahmebüchse /8/ des Blut-Einlasskanals /6/ unterm Neigungswinkel /ϕ/ aus dem Bereich von 15° bis 25° geneigt.

2. Blutpumpe nach Anspruch 1 **gekennzeichnet dadurch, dass** die Randlinie /12/ des Blut-Einlasskanals /6/ tangential am inneren Umfang der Blutkammer /3/ anliegt, hingegen aber das Blut-Auslasskanal /7/ die Form eines unsymetrischen Trichters aufweist, der sich zur Aufnahmebüchse /9/ des Blut-Auslasskanals /7/ hin verjüngt.

3. Blutpumpe nach Anspruch 1 oder 2 **gekennzeichnet dadurch, dass** die Achse /10/ der Aufnahmebüchse /8/ des Blut-Einlasskanals /6/ zu der durch die Basis /5/ der Blutkammer /3/ bestimmten Ebene unterm Neigungswinkel /α/ von weniger als 4°, und vorteilhaft 2°, verläuft.

4. Blutpumpe nach einem der obigen Ansprüche **gekennzeichnet dadurch, dass** die Achse /11/ der Aufnahmebüchse /9/ des Blut-Auslasskanals /7/ zu der durch die Basis /5/ der Blutkammer /3/ bestimmten Ebene unterm Neigungswinkel /β/ aus dem Bereich von 34° bis 37°, und am günstigsten von 35°, verläuft.

5. Blutpumpe nach einem der obigen Ansprüche **gekennzeichnet dadurch, dass** die Achse /11/ der Aufnahmebüchse /9/ des Blut-Auslasskanals /7/ zu der Achse /10/ der Aufnahmebüchse /8/ des Blut-Einlasskanals /6/ im Aufriss auf die Senkrechte zu der durch die Basis /5/ der Blutkammer /3/ bestimmten Ebene und senkrechten zum Aufriss der Achse /10/ der Aufnahmebüchse /8/ des Blut-Einlasskanals /6/ auf die durch die Basis /5/ der Blutkammer /3/ bestimmte Ebene unterm Neigungswinkel /γ/ aus dem Bereich von 25° bis 29°, und am günstigsten von 27°, verläuft.

6. Blutpumpe nach einem der obigen Ansprüche **gekennzeichnet dadurch, dass** die Achse /11/ der Aufnahmebüchse /9/ des Blut-Auslasskanals /7/ zur Achse /10/ der Aufnahmebüchse /8/ des Blut-Einlaufkanals /6/ im Aufriss auf die durch die Basis /5/ der Blutkammer /3/ bestimmte Ebene unterm gewählten Neigungswinkel /ϕ/ aus dem Bereich von 18° bis 22°, und am günstigsten von 20°, verläuft.

## Revendications

1. Pompe à sang, en particulier la chambre d'assistance cardiaque implantable, à commande pneumatique (1), munie d'un boîtier (2), dans lequel on peut distinguer une chambre à air (4) et une chambre à sang (3), séparées par une membrane fixée autour de la circonférence interne du boîtier (2), lequel boîtier définit la base (5) de la chambre à sang (3), où la chambre à sang (3) du boîtier (2) a un canal d'entrée (6) pour le sang et un canal de sortie (7) pour le sang, et dans chaque canal (6, 7) est localisé un logement rond (8, 9) pour y mettre une valvule dans l'objectif de régler le flux de sang à travers la pompe (1), étant donné que pour chaque logement (8, 9) son axe (10, 11) perpendiculaire à la surface définie par l'ouverture de ce logement (8, 9) est incliné sous un angle vers le boîtier (2) de la pompe (1) par rapport à la surface définie par la base (5) de la chambre à sang (3), **caractérisée en ce que** l'axe (10) du logement (8) du canal d'entrée (6) est incliné par rapport au plan défini par la base (5) de la chambre à sang (3) sous l'angle (α) inférieur à 10°, et l'axe (11) du logement (9) du canal de sortie (7) est incliné par rapport au plan défini par la base (5) de la chambre à sang (3) sous un angle (ß) sélectionné dans la gamme de 30 ° à 40 °, et en projection sur le plan perpendiculaire au plan défini par la base (5) de la chambre à sang (3), et perpendiculaire à la projection de l'axe (10) du logement (8) du canal d'entrée (6) sur le plan défini par la base (5) de la chambre à sang (3), l'axe (11) du logement (9) du canal de sortie (7) est incliné par rapport à l'axe (10) du logement (8) du canal d'entrée (6) sous un angle (γ) sélectionné dans la gamme de 22° à 32°, et en projection sur le plan perpendiculaire défini par la base (5) de la chambre à sang (3) l'axe (11) du logement (9) du canal de sortie (7) est incliné à l'axe (10) du logement (8) du canal d'entrée (6) sous un angle () sélectionné dans la gamme de 15°à 25°.

2. Pompe selon la revendication 1, **caractérisée en ce que** la ligne extrême (12) définissant le parcours du canal d'entrée (6) est tangente à la circonférence de l'intérieur de la chambre à sang (3), et le canal de sortie (7) a la forme d'entonnoir asymétrique se rétrécissant en direction du logement (9) du canal de sortie (7).

3. Pompe selon les revendications 1 ou 2, **caractérisée en ce que** l'axe (10) du logement (8) du canal d'entrée (6) est incliné par rapport au plan défini par la base (5) de la chambre à sang (3) sous l'angle (α) inférieur à 4 °, de préférence sous l'angle de 2 °.

4. Pompe selon l'une des revendications mentionnées ci-haut, **caractérisée en ce que** l'axe (11) du logement (9) du canal de sortie (7) est incliné par rapport au plan défini par la base (5) de la chambre à sang (3) sous un angle (ß) sélectionné dans la gamme de 34 ° à 37 °, de préférence sous l'angle de 35 °.

5. Pompe selon l'une des revendications mentionnées ci-haut, **caractérisée en ce qu'**en projection sur le plan perpendiculaire au plan défini par la base (5) de la chambre à sang (3) et perpendiculaire à la projection de l'axe (10) du logement (8) du canal d'entrée (6) sur le plan défini par la base (5) de la chambre à sang (3) l'axe (11) du logement (9) du canal de sortie (7) est incliné par rapport à l'axe (10) du logement (8) du canal d'entrée (6) sous un angle (γ) sélectionné dans la gamme de 25 ° à 29 °, de préférence sous l'angle de 27 °.

6. Pompe selon l'une des revendications mentionnées ci-haut, **caractérisée en ce qu'**en projection sur le plan défini par la base (5) de la chambre à sang (3) l'axe (10) du logement (9) du canal de sortie (7) est incliné par rapport à l'axe (10) du logement (8) du canal d'entrée (6) sous un angle () sélectionné dans la gamme de 18 ° à 22 °, de préférence sous l'angle de 20 °.
